# EUROPÄISCHE PATENTSCHRIFT

(11) **EP 3 440 048 B1**
(45) Veröffentlichungstag und Bekanntmachung des Hinweises auf die Patenterteilung: **29.01.2020**
(21) Anmeldenummer: 17714775.8
(22) Anmeldetag: 31.03.2017
(51) Int. Cl.: C07C 67/055, C07C 67/54, C07C 69/15

(54) **VERFAHREN ZUR HERSTELLUNG VON VINYLACETAT**
PROCESS FOR PREPARING VINYL ACETATE
PROCÉDÉ DE PRÉPARATION D'ACÉTATE DE VINYLE

(30) Priorität: 04.04.2016 DE 102016205487
(43) Veröffentlichungstag der Anmeldung: 13.02.2019
(73) Patentinhaber: Wacker Chemie AG, 81737 München (DE)
(72) Erfinder: WESTERMAYER, Heribert, 84489 Burghausen (DE); MICHL, Harald, 84453 Mühldorf (DE); WAGNER, Johann, 84508 Burgkirchen a. d. Alz (DE)
(74) Vertreter: Schuderer, Michael
(86) Internationale Anmeldenummer: PCT/EP2017/057735
(87) Internationale Veröffentlichungsnummer: WO 2017/174463

(56) Entgegenhaltungen:
- EP-A1- 1 760 065
- DE-A1- 3 422 575
- DE-A1-102013 205 492

## Beschreibung

Die Erfindung betrifft ein Verfahren zur Herstellung von Vinylacetat mittels heterogen katalysierter, kontinuierlicher Gasphasenreaktion von Ethylen, Essigsäure und Sauerstoff in einem Reaktor, wobei das den Reaktor verlassende Produktgemisch mittels Destillation aufgetrennt wird.

Vinylacetat-Monomer (VAM) kann in einem kontinuierlichen Verfahren unter Rückführung des gereinigten Produktstromes hergestellt werden (Kreisgas-Prozess). Dabei reagiert in einer heterogen katalysierten Gasphasenreaktion Ethylen mit Essigsäure und Sauerstoff an Katalysatoren, welche im Allgemeinen Palladium- und Alkalimetallsalze auf einem Trägermaterial enthalten und zusätzlich noch mit Gold oder Rhodium dotiert sein können. Bevorzugt wird ein Pd/Au-Katalysatorgemisch mit Kaliumacetat-Promotor eingesetzt.

Die Edukte Ethylen, Sauerstoff und Essigsäure werden in einer exothermen Reaktion (VAM: Δ_{B}H°₂₉₉ = - 176 kJ/mol), im Allgemeinen bei einem Überdruck von 7 bis 15 bar und, je nach Laufzeit des Katalysators, bei einer Temperatur von im Allgemeinen von 130°C bis 200°C, in einem Festbettröhrenreaktor, aber auch Fluidbettreaktoren, zu Vinylacetat umgesetzt:

C₂H₄ + CH₃COOH + ½O₂ -> CH₃COOCH=CH₂ + H₂O

Hauptnebenreaktion ist dabei die Ethylen-Totaloxidation zu CO₂:

C₂H₄ + 3O₂ -> 2CO₂ + 2H₂O

Der Ethylenumsatz liegt im Allgemeinen bei etwa 5 % bis 20 %, der Essigsäureumsatz bei 20 % bis 60 % und der Sauerstoffumsatz bei bis zu 90 %.

Wegen des unvollständigen Umsatzes von Ethylen und der Nebenreaktionen wird bei der Herstellung von Vinylacetat ein überwiegend aus Ethylen, Kohlendioxid, Ethan, Stickstoff und Sauerstoff bestehendes Gasgemisch (= Kreisgas) im Kreis geführt. Das Kreisgas wird vor dem Festbettröhrenreaktor mit den Edukten Essigsäure, Ethylen und Sauerstoff versetzt und mittels mit Heizdampf betriebenen Wärmetauschern auf Reaktionstemperatur gebracht. Die Anreicherung des Kreisgases mit Essigsäure erfolgt üblicherweise mit einem mit Heizdampf geheizten Essigsäuresättiger.

Nach der Reaktion werden die Reaktionsprodukte Vinylacetat und Wasser und nicht umgesetzte Essigsäure, vorzugsweise in einer sogenannten Vorentwässerungskolonne, aus dem Kreisgas auskondensiert und der weiteren Aufarbeitung zugeführt. Nicht auskondensiertes Edukt/Produkt, im Wesentlichen Ethylen, CO₂ und Vinylacetat, kann am Kopf der Vorentwässerungskolonne entnommen werden und in einem mit Essigsäure betriebenen Wäscher (Kreisgaswäscher) kann das Vinylacetat ausgewaschen werden. Danach kann das so behandelte Kopfprodukt der Vorentwässerungskolonne, das Kreisgas, oder zumindest eine Teilmenge davon, in einem CO₂-Wäscher vom gebildeten Kohlendioxid gereinigt werden. Das Kreisgas wird gegebenenfalls verdichtet, erneut mit den Edukten versetzt, und in den Reaktor zur Gasphasenoxidation geleitet.

Zur weiteren Aufarbeitung des Sumpfprodukts aus der Vorentwässerungskolonne und des Sumpfprodukts der Kreisgaswäsche können die auskondensierten Produkte Vinylacetat und Wasser sowie nicht umgesetzte Essigsäure in einem mehrstufigen, üblicherweise mit Heizdampf betriebenem, Destillationsprozess voneinander getrennt werden. Die üblichen Destillationsschritte zur Gewinnung des Vinylacetats und der Essigsäure sind Azeotropkolonne, Entwässerungskolonne, Rein-VAM-Kolonne, sowie Kolonnen zur Rückstandsaufarbeitung und zur Leichtsieder- und Hochsieder-Abtrennung.

In der DE AS 1278430 wird ein Verfahren beschrieben, bei dem das den Reaktor verlassende Produktgemisch in einer mit Sattelkörpern gefüllten Destillationskolonne aufgetrennt wird. Am Sumpf der Kolonne wird ein Vinylacetat-freies Essigsäure/Wasser-Gemisch abgezogen. Das am Kopf der Kolonne anfallende Destillat wird kondensiert und in eine wässerige und in eine organische Phase getrennt, wobei die aus Vinylacetat, Wasser, Essigsäure und Acetaldehyd bestehende organische Phase in die Destillationskolonne zurückgeführt wird. Nachteilig bei diesem Verfahren ist der relativ hohe Essigsäure-Anteil im Kopfprodukt der Kolonne, welcher die Phasentrennung von Vinylacetat und Wasser erschwert, und deshalb eine zusätzliche destillative Trennung von Vinylacetat und Essigsäure sowie von Essigsäure und Wasser erforderlich macht.

Das Verfahren aus der DE PS 2610624 betrifft die Vorentwässerung des bei der Vinylacetat-Synthese gebildeten Reaktionsgemisches. Bei diesem Verfahren wird das Produktgemisch in eine Vorentwässerungskolonne geleitet. Aus dem Sumpf der Kolonne wird ein Gemisch aus Vinylacetat, Wasser und Essigsäure abgezogen. Das gasförmige Kopfprodukt wird kondensiert und nach Phasentrennung des Kondensats die organische Phase nach Aufheizung in einem Wärmetauscher in die Vorentwässerungskolonne zurückgeführt und die wässrige Phase zur Abwasseraufarbeitung gebracht. Nachteilig bei diesem Verfahren ist die hohe Temperatur mit welcher das gasförmige Reaktionsgemisch in die Vorentwässerungskolonne überführt wird. Messungen haben ergeben, dass bei höheren Eintrittstemperaturen die Kolonne überraschenderweise höhere Ethylacetatwerte am Kopf der Kolonne aufweist, was die Abtrennung von Ethylacetat erschwert. Dieses muss dann aufwändig aus dem Sumpf des Kreisgaswäschers abdestilliert werden. Auch in dem Verfahren gemäß DE 3422575 A1 wird wie in der DE PS 2610624 das den Reaktor verlassende Gasgemisch ohne vorherige Kühlung in eine Vorentwässerungskolonne geleitet, mit den eben beschriebenen Nachteilen.

In der EP 0423658 A2 wird ein Verfahren beschrieben, bei dem das den Reaktor verlassende Produktgasgemisch mit oder ohne Abkühlung der Vorentwässerungskolonne aufgegeben wird. Das Verfahren befasst sich vor allem mit der destillativen Auftrennung des Sumpfproduktes des Kreisgaswäschers in einer im Vergleich zum DE 3422575 A1 zusätzlichen Kolonne. Der Vorteil des Verfahrens ist, dass eine geringere Anzahl an Kolonnenböden in der destillativen Aufarbeitung erforderlich ist.

In der EP 1760065 A1 wird das den Reaktor verlassende Gasgemisch in eine Vorentwässerungskolonne geleitet, welche als Bodenkolonne konstruiert ist. Es wird ein Verfahren beschrieben bei welchem ein Teil des Sumpfprodukts aus dem Kreisgaswäscher in die Vorentwässerungskolonne zurückgeführt wird.

Bei dem in der WO 2014/036132 A1 beschriebenen Verfahren wird der Produktgasstrom aus dem Reaktor in eine Vorentwässerungskolonne geführt. Der Kolonne wird ein Produktgasstrom entnommen, dessen Zusammensetzung sich von dem zugeführten Produktgasstrom unterscheidet, und dieser einem zweiten Reaktor zur Gasphasenoxidation zugeführt. Als Vorentwässerungskolonne werden sowohl Bodenkolonnen als auch Füllkörperkolonnen empfohlen.

Der vorliegenden Erfindung lag die Aufgabe zugrunde, die Aufarbeitung des nach der Gasphasenoxidation erhaltenen Produktgemisches zu optimieren.

Gegenstand der Erfindung ist ein Verfahren zur Herstellung von Vinylacetat mittels heterogen katalysierter, kontinuierlicher Gasphasenreaktion von Ethylen, Essigsäure und Sauerstoff in einem Reaktor, wobei das den Reaktor verlassende Produktgemisch mittels Destillation aufgetrennt wird, wobei die Reaktionsprodukte Vinylacetat und Wasser und nicht umgesetzte Essigsäure aus dem Produktgemisch (Kreisgas) zumindest teilweise auskondensiert werden, und das so behandelte Kreisgas wieder in den Reaktor zurückgeführt wird, dadurch gekennzeichnet, dass
a) das den Reaktor verlassende, gasförmige Produktgemisch mit einem oder mehreren Wärmetauschern auf eine Temperatur von 100°C bis 120°C abgekühlt wird,
b) das damit erhaltene Produktgemisch einer Destillationskolonne aufgegeben wird, welche mit Füllkörpern ausgerüstet ist (Vorentwässerungskolonne), und einen Durchmesser von 2 m bis 6 m hat und mit einer bis vier Packungen oder einer bis vier Schüttungen, mit einer Höhe von jeweils 2 m bis 12 m, ausgerüstet ist,
c) das im wesentlichen Wasser, Vinylacetat und Ethylen enthaltende Kopfprodukt der Vorentwässerungskolonne abgekühlt wird und das dabei anfallende Kondensat (Kopfproduktkondensat) in einem Phasentrenner in eine Wasserphase und eine im wesentlichen Vinylacetat enthaltende organische Phase (Vinylacetat-Phase) aufgetrennt wird,
d) die im Produktgemisch enthaltene Essigsäure zu mindestens 99 Gew.-% über den Sumpf der Vorentwässerungskolonne abgetrennt wird und das Kopfprodukt der Vorentwässerungskolonne <0,1 Gew.-% Essigsäure enthält, gemessen in der Wasserphase des Kopfproduktkondensats,
e) das im Produktgemisch enthaltene Ethylacetat in einer solchen Menge über den Sumpf der Vorentwässerungskolonne abgetrennt wird, dass das Kopfprodukt der Vorentwässerungskolonne < 600 Gew.-ppm Ethylacetat enthält, gemessen in der Vinylacetat-Phase des Kopfproduktkondensats,
f) die Vinylacetat-Phase zu 70 bis 100 Gew.-% in die Vorentwässerungskolonne zurückgeführt wird, und
g) am Sumpf der Vorentwässerungskolonne ein Produktgemisch abgezogen wird, welches 10 bis 60 Gew.-% Vinylacetat enthält.

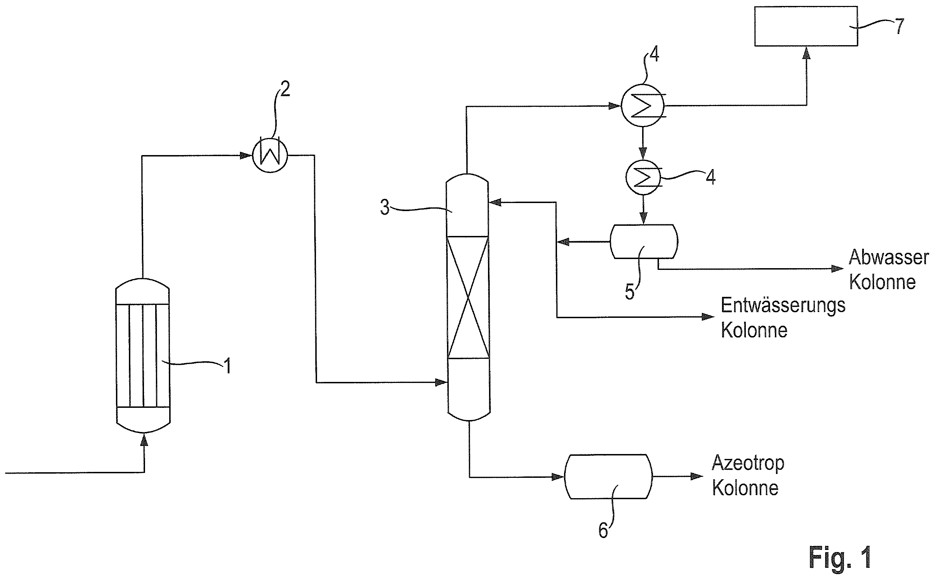

Das erfindungsgemäße Verfahren wird nachfolgend und in Figur 1 näher erläutert. Die Anordnung der Apparaturen zur Auftrennung des Produktgemisches aus der Gasphasenreaktion zur Herstellung von Vinylacetat ist vielfach beschrieben und dem Fachmann bekannt. Diese kann beispielsweise auch der WO 2015/082450 A1 entnommen werden.

Zur Herstellung von Vinylacetat mittels heterogen katalysierter, kontinuierlicher Gasphasenreaktion von Ethylen, Essigsäure und Sauerstoff wird das sogenannte Kreisgas mit Frischethylen beladen, welches den bei der Reaktion verbrauchten Ethylenanteil ersetzt, und einem Essigsäuresättiger zugeführt. Im Essigsäuresättiger, im Allgemeinen eine Kolonne mit Füllkörpern oder mit Böden, wird die bei der Reaktion verbrauchte und im Kreisgas auskondensierte Essigsäure durch Einspeisung von Frischessigsäure und Rückessigsäure ersetzt. Dem mit Ethylen und Essigsäure beladenen Kreisgas wird vor dem Eintritt in den Reaktor über eine Düse Sauerstoff zugegeben.

Anschließend wird das Kreisgas mit einem Kreisgasdruck von vorzugsweise 7 bis 15 bar abs. in den Reaktor (1) geleitet, vorzugsweise einem Festbett-Rohrbündelreaktor aus einem zylinderförmigen Behälter, in welchem mehrere tausend, üblicherweise 2000 bis 20000, dicht gepackte zylinderförmige Reaktionsrohre angeordnet sind. Für den großtechnischen Einsatz werden dazu Rohre mit einer Länge von vorzugsweise 5 bis 10 m und einem inneren Durchmesser von vorzugsweise 25 bis 40 mm eingesetzt. Die Zwischenräume zwischen den Rohren und den Rohren und der Behälterwand werden zur Kühlung von einem Wasser/Wasserdampf-Gemisch als Wärmetauschmedium durchströmt.

Die Rohre sind mit Trägerkatalysatoren befüllt, basierend auf einem inerten, anorganischen Trägermaterial wie Titanoxide, Siliciumoxiden, Aluminiumoxiden, welche mit einer Palladiumverbindung in Kombination mit Alkalimetallen beschichtet sind, und zusätzlich noch mit Gold, Rhodium oder Cadmium dotiert sein können. Diese Trägerkatalysatoren können in Form von Kugeln, Zylindern oder Ringen vorliegen, wobei deren Abmessungen den Rohren angepasst sind.

Die Reaktion erfolgt im Allgemeinen bei einem Druck von vorzugsweise 7 bis 15 bar abs. und bei einer Temperatur von im Allgemeinen von 130°C bis 200°C.

Das den Reaktor (1) verlassende, gasförmige Produktgemisch, welches im Wesentlichen Ethylen, Vinylacetat, Essigsäure, Wasser, Kohlendioxid, Ethylacetat, Sauerstoff und Inerten wie Argon und Stickstoff enthält, wird vor dessen Eintritt in die Vorentwässerungskolonne mit einem oder mehreren Wärmetauschern (2) auf eine Temperatur von 100°C bis 120°C, vorzugsweise 105°C bis unter 115°C, abgekühlt. Dabei kann bereits Kondensat entstehen.

Das abgekühlte Produktgemisch wird einer Destillationskolonne (3) aufgegeben, welche anstelle von Böden mit Füllkörpern ausgerüstet ist (Vorentwässerungskolonne). Als Füllkörper können die in der chemischen Verfahrenstechnik gängigen Füllkörper eingesetzt werden. Beispielsweise Füllkörper aus metallischen oder keramischen Materialien oder aus Kunststoff. Bevorzugt sind metallische Materialien. Beispiele für metallische Materialien sind Eisen, wie Stahl, insbesondere Edelstahl, Kupfer, Messing, Aluminium, Nickel, Monel-Metalle oder Titan. Beispiele für keramische Materialien sind Oxide der Hauptgruppen-Metalle oder -Halbmetalle, beispielsweise von Bor, Aluminium oder Silicium, insbesondere Borsilikatgläser oder Aluminiumsilikatgläser. Beispiele für Kunststoffe sind Polyolefine, Halogen substituierte Polyolefine, Polyethersulfone, Polyphenylensulfide oder Polyaryletherketone. Die Füllkörper können in unterschiedlichsten Formen vorliegen, wie beispielsweise in Hohlzylinder-Form, auch Ringe genannt, Sattel- oder Kugel-Form. Bevorzugt sind Pall-Ringe, Berl-Sattel, Hiflow-Ringe, Intalox-Sattel, Igel oder insbesondere Raschig-Super-Ringe. Die Füllkörper haben einen oder mehrere Durchmesser von vorzugsweise 5 bis 100 mm, besonders bevorzugt 10 bis 80 mm und am meisten bevorzugt 25 bis 50 mm. Die Füllkörper haben eine spezifische Oberfläche von vorzugsweise 80 bis 350 m²/m³ und besonders bevorzugt von 100 bis 250 m²/m³. Die spezifische Oberfläche errechnet sich hierbei durch Multiplizieren der Oberfläche des Materials, aus dem ein Füllkörper geformt ist, mit der Stückzahl dieser Füllkörper, bezogen auf einen Kubikmeter der Schüttung.

Die Füllkörper können als Packung oder vorzugsweise als Schüttung eingesetzt werden. In einer Schüttung liegt bekanntermaßen eine Vielzahl von Füllkörpern in loser und ungeordneter Schichtung auf Trageelementen, wie perforierte Trageroste oder sonstige Auflageböden oder Roste. Zur Fixierung der Füllkörperschüttungen kann die Kolonne am oberen Ende der Schüttung jeweils mit einem Niederhalterost ausgerüstet werden. Bei Einsatz von mehreren Trageelementen können mehrere Schichten von Füllkörpern eingebaut werden (Schichtungen). Packungen weisen eine regelmäßig geformte Struktur auf, wie beispielsweise Gewebe- oder Blechpackungen, insbesondere dünne, gewellte oder gelochte Platten bzw. Netze, beispielsweise aus Metall, Kunststoff, Glas oder Keramik. Das jeweilige Prozessgas strömt wie üblich durch die Schüttung bzw. Packung hindurch. Die Packungen haben eine spezifische Oberfläche von vorzugsweise 100 bis 750 m²/m³ und besonders bevorzugt von 150 bis 350 m²/m³. Die spezifische Oberfläche errechnet sich hierbei aus der Oberfläche des Materials, aus dem die Packung geformt wird, bezogen auf einen Kubikmeter der Packung. Vorzugsweise werden keine Goodloe-Packungen eingesetzt, das sind Packungen aus gerolltem VA-Drahtnetz.

Erfindungsgemäß hat die Vorentwässerungskolonne (3) im großtechnischen Maßstab einen Durchmesser von 2 m bis 6 m, vorzugsweise 3 m bis 5 m. Erfindungsgemäß ist die Vorentwässerungskolonne (3) mit einer bis vier Packungen oder einer bis vier Schüttungen, vorzugsweise zwei bis drei Packungen oder zwei bis drei Schüttungen, mit einer Höhe von jeweils 2 m bis 12 m, vorzugsweise 5 m bis 9 m, ausgerüstet.

Vorzugsweise arbeitet man bei der Vorentwässerungskolonne mit einer theoretischen Trennstufenzahl für die Essigsäure- und Ethylacetatabtrennung von mindestens 5, vorzugsweise von mindestens 10, noch mehr bevorzugt von mindestens 13, wobei hier bei der Höhenauslegung der Kolonne zu beachten ist, dass durch den hohen Inertgasanteil in der Gasphase (Ethylen), die HETP-Werte stark erhöht sind (HETP = Height Equivalent to a Theoretical Plate).

Die Vorentwässerungskolonne (3) kann beheizt werden und wird vorzugsweise ohne zusätzliche Heizung betrieben. Am Kopf der Vorentwässerungskolonne (3) wird das im wesentlichen Wasser, Vinylacetat und Ethylen enthaltende Kopfprodukt mittels Wärmetausch in einem oder mehreren Wärmetauschern (4) auf vorzugsweise 10°C bis 35°C, besonders bevorzugt 10°C bis 30°C, abgekühlt. Das dabei anfallende Kondensat (Kopfproduktkondensat) kann dabei in einem eigenen Wärmetauscher (4) abgekühlt werden und wird in einem Phasentrenner (5) in eine Wasserphase und eine im wesentlichen Vinylacetat enthaltende organische Phase (Vinylacetat-Phase) aufgetrennt.

Die Kolonne wird so betrieben, dass die im Produktgemisch enthaltene Essigsäure zu mindestens 99 Gew.-%, vorzugsweise zu 99,9 bis 100 Gew.-%, über den Sumpf der Vorentwässerungskolonne (3) abgetrennt wird, und dass das Kopfprodukt der Vorentwässerungskolonne < 0,1 Gew.-% Essigsäure, vorzugsweise < 0,01 Gew.-% Essigsäure, am meisten bevorzugt 20 bis 50 Gew.-ppm Essigsäure enthält, jeweils gemessen in der Wasserphase des Kopfproduktkondensats, das aus dem Phasentrenner (5) abgezogen wird.

Das im Produktgemisch enthaltene Ethylacetat wird in einer solchen Menge über den Sumpf der Vorentwässerungskolonne (3) abgetrennt, dass das Kopfprodukt der Vorentwässerungskolonne < 600 Gew.-ppm Ethylacetat, vorzugsweise 100 bis 300 Gew.-ppm enthält, jeweils gemessen in der Vinylacetat-Phase des Kopfproduktkondensats.

Die Vinylacetat-Phase kann mit oder ohne weitere Aufheizung mittels einem oder mehreren Wärmetauschern, zum Beispiel im Wärmeverbund mit dem aus der Kolonne austretendem Brüdenstrom, in die Vorentwässerungskolonne (3) zurückgeführt werden. Vorzugsweise wird die Vinylacetat-Phase ohne weitere Aufheizung in die Vorentwässerungskolonne (3) zurückgeführt. Vorzugsweise wird die flüssige Vinylacetat-Phase am Kopf der Vorentwässerungskolonne (3) aufgegeben. Die Trennleistung der Vorentwässerungskolonne (3) wird in dem Fachmann bekannter Weise über die Temperatur des Produktgemisches beim Eintritt in die Vorentwässerungskolonne (3), über die Kreisgasaustrittstemperatur nach der Kondensation mit den Wärmetauschern (4) und über die Menge an Rücklauf der Vinylacetat-Phase aus dem Phasentrenner (5) in die Vorentwässerungskolonne (3) gesteuert. Im Allgemeinen wird die Vinylacetat-Phase zu 70 bis 100 Gew.-%, vorzugsweise zu 85 bis 100 Gew.-% in die Vorentwässerungskolonne (3) zurückgeführt. Der Restanteil kann gegebenenfalls zu einer Entwässerungskolonne abgegeben werden, in welcher das Vinylacetat/Wasser-Gemisch aus der AzeotropKolonne aufgetrennt wird.

Die nicht kondensierten Anteile des Kopfprodukts der Vorentwässerungskolonne (3), im Wesentlichen Ethylen, Kohlendioxid und Vinylacetat werden zum Kreisgaswäscher (7) abgegeben. Dabei bestimmt die Kreisgasaustrittstemperatur aus den Wärmetauschern (4) über den Taupunkt die Menge an Vinylacetat, die zum Kreisgaswäscher geht. Dort werden die nicht kondensierten Vinylacetat-Anteile mit Essigsäure absorbiert und aus dem Kreisgas entfernt.

Danach kann das Kreisgas, oder zumindest eine Teilmenge davon, in einem CO₂-Wäscher (CO₂-Absorption/Desorption) von gebildetem Kohlendioxid gereinigt werden. Die zur Kreisgaswäsche eingesetzte Essigsäure kann beispielsweise von der Azeotropkolonne und/oder aus der Rückstandsaufarbeitung zugeführt werden. Das Sumpfprodukt aus dem Kreisgaswäscher (7) kann gegebenenfalls ganz oder teilweise in die Vorentwässerungskolonne (3) zurückgeführt werden. Der Essigsäure-haltige Wasserstrom aus einem Wasserwäscher vor der CO₂-Wäsche (CO₂-Absorption/Desorption) kann gegebenenfalls ganz oder teilweise in die Vorentwässerungskolonne (3) zurückgeführt werden. Das nach KreisgasWäscher und CO₂-Wäscher nun Vinylacetat-freie Kreisgas kann über einen Kreisgasverdichter und einen Essigsäure-Sättiger wieder der Reaktion im Reaktor zugeführt werden.

Das Sumpfprodukt der Vorentwässerungskolonne (3), welches 10 bis 60 Gew.-%, vorzugsweise 30 bis 50 Gew.-%, Vinylacetat enthält, kann in einem Sammelbehälter (6), dem Rohvinylacetatbehälter, gesammelt werden und in einer weiteren Destillationskolonne, der Azeotropkolonne, weiter aufgetrennt werden. In der Azeotropkolonne erhält man im Sumpf die Essigsäure, welche als Rückessigsäure dem Kreisgas wieder aufgegeben werden kann. Über einen Seitenabzug kann das Ethylacetat zur Ethylacetatkolonne, zur Entfernung des Ethylacetats, ausgeschleust werden. Am Kopf der Azeotropkolonne wird ein Wasser und Vinylacetat enthaltendes Gemisch erhalten, welches in der Entwässerungskolonne aufgetrennt werden kann.

Nach Phasentrennung in eine Wasserphase und eine Vinylacetat-Phase kann das Vinylacetat in einer Entwässerungs-Kolonne entwässert werden und in einer Rein-Vinylacetat-Kolonne gewonnen werden. Die wässrige Phase kann zusammen mit weiteren Wasserphasen aus dem Prozess, beispielsweise der Wasserphase aus dem Phasentrenner (5) hinter der Vorentwässerungskolonne (3), in einer Abwasserkolonne behandelt werden.

Überraschenderweise blieb mit der Umrüstung von Böden auf Füllkörper (Revamp), d.h. mit gleichem Kolonnendurchmesser und gleicher Kolonnenhöhe, die Trennleistung der Vorentwässerungskolonne (3) ungefähr gleich. Dies war deshalb nicht zu erwarten, da in der Vorentwässerungskolonne (3) zwei flüssige Phasen miteinander in Kontakt treten, eine Vinylacetat-Essigsäure-Phase und eine Wasserphase. Bei zwei Flüssigphasen der sogenannten Dreiphasenrektifikation wird im Allgemeinen erwartet, dass in einer Bodenkolonne, wegen der dort auftretenden permanenten Durchmischung der Flüssigphasen, die Trennleistung besser wäre. Deshalb werden in der Industrie im Bereich der Dreiphasenrektifikation überwiegend Bodenkolonnen eingesetzt. Packungs- und Füllkörperkolonnen finden in diesem Bereich nicht gezielt Anwendung, da das Trennleistungsverhalten (Trennleistungsabfall durch Entmischung der 2 flüssigen Phasen) und der Umgang mit den zwei flüssigen Phasen in den Verteilern ungeklärt ist.

Mit der Umrüstung (Revamp) einer Bodenkolonne als Vorentwässerungskolonne (3) auf eine Füllkörperkolonne konnte im großtechnischen Maßstab bei gleichem Kolonnendurchmesser und gleicher Kolonnenhöhe die Kreisgasmenge einer Anlage um 25 % gesteigert werden, gemessen vor dem Essigsäuresättiger und nach der Ethylenzugabe, und die Kapazität einer Anlage um fast 20 % von 170000 Tonnen pro Jahr auf 200000 Tonnen pro Jahr Vinylacetat gesteigert werden, da die vorzeitige Flutung der Bodenkolonne (vermutlich aufgrund Schaumbildung) ab einer bestimmten Kreisgasmenge, mittels der Ausrüstung mit Füllkörpern verhindert werden konnte.

Ein weiterer Vorteil war der, dass die Verweilzeit von Vinylacetat in der Kolonne reduziert wird, da wesentlich weniger Flüssigkeitsholdup in der Kolonne vorhanden ist, und damit auch der Abbau von Vinylacetat wegen Rückreaktion (Hydrolyse zu Acetaldehyd und Essigsäure) reduziert wird. Es resultierte eine Steigerung der Ausbeute an Vinylacetat um 1 Gew.-% bei gleichem Edukteinsatz. Bei einer großtechnischen Anlage mit einer Kapazität von 200000 Tonnen pro Jahr Vinylacetat ist das ein Plus von 2000 Tonnen Vinylacetat pro Jahr.

Mit dem erfindungsgemäßen Verfahren, bei welchem das den Reaktor verlassende Produktgemisch vor dem Eintritt in die Vorentwässerungskolonne (3) abgekühlt wird, erhöhte sich auch der Anteil von Vinylacetat im Sumpf der Vorentwässerungskolonne (3). Überraschenderweise konnte bei den aus dem Stand der Technik bekannten Verfahren beobachtet werden, dass mehr Vinylacetat zum Kreisgaswäscher (7) geht, als nach dem Taupunkt möglich wäre. Es wird eine Aerosolproblematik am Kopf der Vorentwässerungskolonne (3) vermutet, wodurch zusätzlich Vinylacetat als feinste Tröpfchen (Aerosole) nach den Wärmeaustauschern (4) zum Kreisgaswäscher (7) verschleppt werden. Mit dem erfindungsgemäßen Verfahren konnte diese Aerosolproblematik reduziert werden, da bei gleicher Kreisgasaustrittstemperatur nach den Wärmeaustauschern (4) weniger Vinylacetat im Kreisgaswäscher (7) ankommt, dafür aber mehr Vinylacetat im Sumpfprodukt der Vorentwässerungskolonne (3) vorhanden ist. Folglich musste weniger Vinylacetat aus der Gasphase im Kreisgaswäscher entfernt werden. Dies führte zu einer deutlichen Entlastung des Kreisgaswäschers.

Es konnte auch der Druckverlust in der Vorentwässerungskolonne mit den Füllkörpern reduziert werden, was wiederum den Kreisgasverdichter entlastet, und dort zu einer deutlichen Reduzierung des Stromverbrauchs führte.

Die Limitierung des Essigsäureanteils im Kopfprodukt der Vorentwässerungskolonne erhält und vertieft die Mischungslücke Wasser und Vinylacetat im Phasentrenner, welche eine scharfe Auftrennung dieser Bestandteile gewährleistet. Die Limitierung des Ethylacetat-Anteils auf < 600 ppm im Kopfprodukt der Vorentwässerungskolonne verhindert den Austrag des Ethylacetats über den Kreisgasstrom, was aufwändige Maßnahmen zur Verhinderung von Ethylacetat im Rein-Vinylacetat vermeiden hilft.

## Patentansprüche

1. Verfahren zur Herstellung von Vinylacetat mittels heterogen katalysierter, kontinuierlicher Gasphasenreaktion von Ethylen, Essigsäure und Sauerstoff in einem Reaktor, wobei das den Reaktor verlassende Produktgemisch mittels Destillation aufgetrennt wird, wobei die Reaktionsprodukte Vinylacetat und Wasser und nicht umgesetzte Essigsäure aus dem Produktgemisch (Kreisgas) zumindest teilweise auskondensiert werden, und das so behandelte Kreisgas wieder in den Reaktor zurückgeführt wird, **dadurch gekennzeichnet, dass**
a) das den Reaktor verlassende, gasförmige Produktgemisch mit einem oder mehreren Wärmetauschern auf eine Temperatur von 100°C bis 120°C abgekühlt wird,
b) das damit erhaltene Produktgemisch einer Destillationskolonne aufgegeben wird, welche mit Füllkörpern ausgerüstet ist (Vorentwässerungskolonne), und einen Durchmesser von 2 m bis 6 m hat und mit einer bis vier Packungen oder einer bis vier Schüttungen, mit einer Höhe von jeweils 2 m bis 12 m, ausgerüstet ist,
c) das im wesentlichen Wasser, Vinylacetat und Ethylen enthaltende Kopfprodukt der Vorentwässerungskolonne abgekühlt wird und das dabei anfallende Kondensat (Kopfproduktkondensat) in einem Phasentrenner in eine Wasserphase und eine im wesentlichen Vinylacetat enthaltende organische Phase (Vinylacetat-Phase) aufgetrennt wird,
d) die im Produktgemisch enthaltene Essigsäure zu mindestens 99 Gew.-% über den Sumpf der Vorentwässerungskolonne abgetrennt wird und das Kopfprodukt der Vorentwässerungskolonne < 0,1 Gew.-% Essigsäure enthält, gemessen in der Wasserphase des Kopfproduktkondensats,
e) das im Produktgemisch enthaltene Ethylacetat in einer solchen Menge über den Sumpf der Vorentwässerungskolonne abgetrennt wird, dass das Kopfprodukt der Vorentwässerungskolonne ≤ 600 Gew.-ppm Ethylacetat enthält, gemessen in der Vinylacetat-Phase des Kopfproduktkondensats,
f) die Vinylacetat-Phase zu 70 bis 100 Gew.-% in die Vorentwässerungskolonne zurückgeführt wird, und
g) am Sumpf der Vorentwässerungskolonne ein Produktgemisch abgezogen wird, welches 10 bis 60 Gew,-% Vinylacetat enthält.

2. Verfahren nach Anspruch 1, **dadurch gekennzeichnet, dass** das Kopfprodukt der Vorentwässerungskolonne < 0,01 Gew.-% Essigsäure, gemessen in der Wasseranphase des Kopfproduktkondensats, enthält.

3. Verfahren nach Anspruch 1 bis 2, **dadurch gekennzeichnet, dass** das Kopfprodukt der Vorentwässerungskolonne auf 10°C bis 35°C abgekühlt wird.

4. Verfahren nach Anspruch 1 bis 3, **dadurch gekennzeichnet, dass** das Kreisgas zu einem Kreisgaswäscher abgegeben wird und das Sumpfprodukt aus dem Kreisgaswäscher ganz oder teilweise in die Vorentwässerungskolonne zurückgeführt wird.

5. Verfahren nach Anspruch 4, **dadurch gekennzeichnet, dass** das Kreisgas nach der Behandlung im Kreisgaswäscher in einem CO₂-Wäscher vom gebildeten Kohlendioxid gereinigt wird und dass der Essigsäure-haltige Wasserstrom aus einem Wasserwäscher vor der CO₂-Wäsche (CO₂-Absorption/Desorption) ganz oder teilweise in die Vorentwässerungskolonne zurückgeführt wird.

## Claims

1. A process for preparing vinyl acetate by means of a heterogeneously catalyzed, continuous gas-phase reaction of ethylene, acetic acid and oxygen in a reactor, where the product mixture leaving the reactor is fractionated by means of distillation, the reaction products vinyl acetate and water and unreacted acetic acid are at least partly condensed out from the product mixture (recycle gas) and the recycle gas which has been treated in this way is recirculated back into the reactor, **characterized in that**
a) the gaseous product mixture leaving the reactor is cooled to a temperature of from 100°C to 120°C by means of one or more heat exchangers,
b) the resulting product mixture is fed to a distillation column which is equipped with packing elements (predewatering column), and has a diameter of from 2 m to 6 m and is equipped with from one to four ordered packings or from one to four beds each having a height of from 2 m to 12 m,
c) the overhead product containing essentially water, vinyl acetate and ethylene from the predewatering column is cooled and the condensate obtained (overhead product condensate) is separated in a phase separator into an aqueous phase and an organic phase containing essentially vinyl acetate (vinyl acetate phase),
d) the acetic acid present in the product mixture is separated off to an extent of at least 99% by weight via the bottom of the predewatering column and the overhead product from the predewatering column contains <0.1% by weight of acetic acid, measured in the aqueous phase of the overhead product condensate,
e) the ethyl acetate present in the product mixture is separated off via the bottom of the predewatering column in such an amount that the overhead product from the predewatering column contains ≤ 600 ppm by weight of ethyl acetate, measured in the vinyl acetate phase of the overhead product condensate,
f) the vinyl acetate phase is recirculated to an extent of from 70 to 100% by weight into the predewatering column and
g) a product mixture containing from 10 to 60% by weight of vinyl acetate is taken off at the bottom of the predewatering column.

2. Process according to Claim 1, **characterized in that** the overhead product from the predewatering column contains < 0.01% by weight of acetic acid, measured in the aqueous phase of the overhead product condensate.

3. Process according to any of Claims 1 to 2, **characterized in that** the overhead product from the predewatering column is cooled to from 10°C to 35°C.

4. Process according to any of Claims 1 to 3, **characterized in that** the recycle gas is passed to a recycle gas scrubber and the bottom product from the recycle gas scrubber is recirculated in its entirety or in part to the predewatering column.

5. Process according to Claim 4, **characterized in that** the recycle gas after the treatment in the recycle gas scrubber is freed of carbon dioxide in a CO₂ scrubber and **in that** the acetic acid-containing water stream from a water scrubber upstream of the CO₂ scrub (CO₂ absorption/desorption) is recirculated in its entirety or in part to the predewatering column.

## Revendications

1. Procédé pour la préparation d'acétate de vinyle au moyen d'une réaction catalysée de manière hétérogène en phase gazeuse en continu d'éthylène, d'acide acétique et d'oxygène dans un réacteur, le mélange de produits quittant le réacteur étant séparé au moyen d'une distillation, les produits de réaction acétate de vinyle et eau et de l'acide acétique qui n'a pas été transformé étant condensés au moins partiellement à partir du mélange de produits (gaz de circulation), et le gaz de circulation ainsi traité étant à nouveau renvoyé dans le réacteur, **caractérisé en ce que**
a) le mélange de produits gazeux quittant le réacteur est refroidi avec un ou plusieurs échangeurs de chaleur à une température de 100 °C à 120 °C,
b) le mélange de produits ainsi obtenu est chargé dans une colonne de distillation, qui est équipée de corps de remplissage (colonne de déshydratation préliminaire), et qui possède un diamètre de 2 m à 6 m et qui est équipé d'un à quatre éléments de garnissage ou équipé d'un à quatre lits, d'une hauteur d'à chaque fois 2 m à 12 m,
c) le produit de tête de la colonne de déshydratation préliminaire contenant essentiellement de l'eau, de l'acétate de vinyle et de l'éthylène est refroidi et le condensat alors obtenu (condensat de produit de tête) est séparé dans un séparateur de phases en une phase aqueuse et une phase organique contenant essentiellement de l'acétate de vinyle (phase acétate de vinyle),
d) l'acide acétique contenu dans le mélange de produits est séparé à raison d'au moins 99 % en poids par l'intermédiaire du collecteur de la colonne de déshydratation préliminaire et le produit de tête de la colonne de déshydratation préliminaire contient < 0,1 % en poids d'acide acétique, mesuré dans la phase aqueuse du condensat de produit de tête,
e) l'acétate d'éthyle contenu dans le mélange de produits est séparé par l'intermédiaire du collecteur de la colonne de déshydratation préliminaire en une telle quantité que le produit de tête de la colonne de déshydratation préliminaire contient ≤ 600 ppm en poids d'acétate d'éthyle, mesuré dans la phase acétate de vinyle du condensat de produit de tête,
f) la phase acétate de vinyle est renvoyée à raison de 70 à 100 % en poids dans la colonne de déshydratation préliminaire, et
g) un mélange de produits est extrait au niveau du collecteur de la colonne de déshydratation préliminaire, qui contient 10 à 60 % en poids d'acétate de vinyle.

2. Procédé selon la revendication 1, **caractérisé en ce que** le produit de tête de la colonne de déshydratation préliminaire contient < 0,01 % en poids d'acide acétique, mesuré dans la phase aqueuse du condensat de produit de tête.

3. Procédé selon les revendications 1 et 2, **caractérisé en ce que** le produit de tête de la colonne de déshydratation préliminaire est refroidi à une température de 10 °C à 35 °C.

4. Procédé selon les revendications 1 à 3, **caractérisé en ce que** le gaz de circulation est chargé dans un laveur de gaz de circulation et le produit de fond du laveur de gaz de circulation est renvoyé totalement ou partiellement dans la colonne de déshydratation préliminaire.

5. Procédé selon la revendication 4, **caractérisé en ce que** le gaz de circulation, après le traitement dans le laveur de gaz de circulation, est épuré du dioxyde de carbone formé dans un laveur de CO₂ et que le flux d'eau contenant de l'acide acétique d'un laveur d'eau est renvoyé avant le lavage du CO₂ (absorption/désorption de CO₂) totalement ou partiellement dans la colonne de déshydratation préliminaire.
